# EUROPEAN PATENT APPLICATION

(11) **EP 4 736 864 A1**
(43) Date of publication of application: **06.05.2026**
(21) Application number: 25212054.8
(22) Date of filing: 29.10.2025
(51) Int. Cl.: A61K 36/899, A61P 1/00, A23L 33/105

(54) **COMPOSITION FOR PREVENTING OR TREATING INFLAMMATORY BOWEL DISEASE COMPRISING A GERMINATED OAT EXTRACT**

(30) Priority: 30.10.2024 KR 20240151416
(71) Applicant: INGR Inc., Andong-si, Gyeongsangbuk-do 36618 (KR)
(72) Inventor: PYEE, Jae Ho, Gunpo-si, Gyeonggi-do 15804 (KR)
(74) Representative: Vossius & Partner Patentanwälte Rechtsanwälte mbB

(57) **Abstract**

The present invention relates to a composition for preventing, alleviating, or treating inflammatory bowel disease, comprising a germinated oat (Avena sativa L.) extract. The composition improves intestinal permeability and enhances tight junction integrity in an inflamed intestinal environment, and thus can be effectively used in the manufacture of a pharmaceutical, health functional food, food or prebiotics for the prevention, alleviation, or treatment of inflammatory bowel disease.

## Description

### [Technical Field]

The present invention relates to a composition for preventing or treating inflammatory bowel disease, comprising a germinated oat extract with an increased avenanthramide content.

### [Background Art]

Inflammatory bowel disease (IBD) refers to a chronic inflammatory disorder in which inflammation or ulcers occur in the intestine due to unknown causes, including ulcerative colitis (UC), Crohn' s disease (CD), and Behcet' s disease.

Patients with inflammatory bowel disease are common worldwide and have shown a markedly increasing trend in recent years in Korea; however, the causes and mechanisms of the disease are relatively complex and have not yet been clearly elucidated.

Conventional drugs used for the treatment of inflammatory bowel disease include steroidal immunosuppressants, 5-aminosalicylic acid (5-ASA) based drugs, such as sulfasalazine, which block the production of prostaglandins, and mesalazine. As exemplified above, anti-inflammatory agents and immunosuppressants are prescribed; however, they are associated with side effects such as nausea, vomiting, headache, hemolytic anemia, and male infertility. In addition, a considerable number of patients either do not respond to the treatment or experience reduced efficacy, and thus there is a continuing demand for the development of safer and more effective therapeutics.

Inflammatory bowel disease is characterized by damage to the integrity of the epithelial barrier caused by inflammation. Increased permeability of the epithelial tight junctions and impaired barrier integrity lead to weakening of the intestinal barrier, which is referred to as a "leaky gut." Recent studies have revealed that the intestinal barrier plays an important role not only in inflammatory bowel disease but also in various diseases, including autoimmune disorders associated with barrier integrity. Maintaining and strengthening barrier integrity is important for the prevention and treatment of inflammatory bowel disease as well as other diverse diseases. Recently, natural bioactive substances known for their anti-inflammatory effects have attracted attention as important potential therapeutics for the treatment of inflammatory bowel disease.

Oat (Avena sativa L.) contains abundant bioactive components and exhibits various health-promoting effects, including antioxidant, antidiabetic, antibacterial, anticancer, antihypertensive, immunomodulatory, antihyperlipidemic, anti-obesity, cardioprotective, and anti-inflammatory effects. According to prior studies, germinated oat has been reported to regulate the expression of junction proteins in the skin, enhance skin barrier function, and treat conditions of skin barrier disruption, thereby exhibiting efficacy against atopic dermatitis.

Oat contains various phenolic compounds, among which avenanthramides (AVNs) are a type of phenolic alkaloid represented by the following Chemical Formula I, and are specifically contained in oat. The contents of avenanthramides and phenolic compounds in germinated oat are significantly higher than those in ungerminated oat seeds.

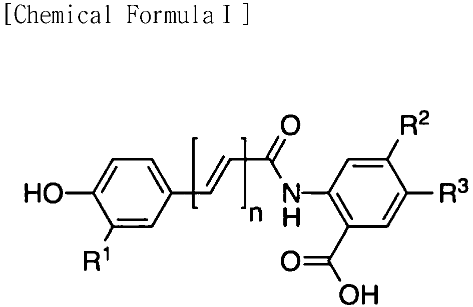

Avenanthramides are known to be potent antioxidants and have been reported to exhibit effects such as blood pressure regulation, inhibition of skin irritation, and anti-atherosclerotic activity. Recently, they have also been found to be useful in the treatment of hearing loss and neurodegenerative diseases. As such, research on the applications of avenanthramides has been actively conducted, and they are attracting attention as valuable components that can be used in the treatment of various diseases.

However, in the context of inflammatory bowel disease (IBD), the specific effects of active components of germinated oat on intestinal permeability and tight junction (TJ) integrity have not been clearly elucidated.

Accordingly, the present inventors conceived inflammatory bowel disease (IBD) as a phenotype associated with the interaction between bioactive compounds of germinated oat and the genetic and proteomic environment determining tight junction integrity, and adopted an integrated approach combining bioinformatics analysis, chemical profiling, and an initial in silico evaluation using the Combination-Oriented Natural Product Database with Unified Terminology (COCONUT). In addition, by focusing on the efficacy evaluation through in silico predictions validated with an in vitro model using Caco-2 cells, the present inventors elucidated the underlying mechanism of the effect of germinated oat on intestinal permeability, and confirmed that a germinated oat extract (GOE) improves tight junction integrity in an inflamed intestinal environment, thereby completing the present invention.

### [Prior Art Documents]

### [Patent Documents]

(Patent Document 0001) Korean Registration Patent No. 10-1745734
(Patent Document 0002) Korean Registration Patent No. 10-1935500

### [Disclosure of the Invention]

### [Problem to be Solved by the Invention]

The object of the present invention, which was conceived to solve the above-described problems, is to provide a pharmaceutical composition for preventing or treating inflammatory bowel disease, comprising a germinated oat (Avena sativa L.) extract.

Another object of the present invention is to provide a health functional food composition for alleviating inflammatory bowel disease, comprising a germinated oat extract.

Still another object of the present invention is to provide a food composition for preventing or alleviating inflammatory bowel disease, comprising a germinated oat extract.

Another object of the present invention is to provide a prebiotic composition for improving intestinal microbiota, comprising a germinated oat extract as an active ingredient.

### [Means for Solving the Problem]

The present invention relates to a composition for preventing, alleviating, or treating inflammatory bowel disease (IBD), comprising a germinated oat (Avena sativa L.) extract. The composition according to the present invention exhibits preventive, alleviative, or therapeutic effects on inflammatory bowel disease by improving intestinal permeability and tight junction integrity in an inflamed intestinal environment.

In one embodiment of the present invention, there is provided a pharmaceutical composition for preventing or treating inflammatory bowel disease, comprising a germinated oat extract.

In another embodiment of the present invention, there is provided a health functional food composition for preventing or alleviating inflammatory bowel disease, comprising a germinated oat extract.

In still another embodiment of the present invention, there is provided a food composition for preventing or alleviating inflammatory bowel disease, comprising a germinated oat extract.

In another embodiment of the present invention, there is provided a prebiotic composition for improving intestinal microbiota, comprising a germinated oat extract as an active ingredient.

### [Effects of the Invention]

The present invention relates to a composition for preventing, alleviating, or treating inflammatory bowel disease, comprising a germinated oat (Avena sativa L.) extract. The composition improves intestinal permeability and enhances tight junction integrity in an inflamed intestinal environment, and thus can be effectively used for the manufacture of a pharmaceutical, health functional food, food or prebiotics for the prevention, alleviation, or treatment of inflammatory bowel disease.

### [Brief Description of Drawings]

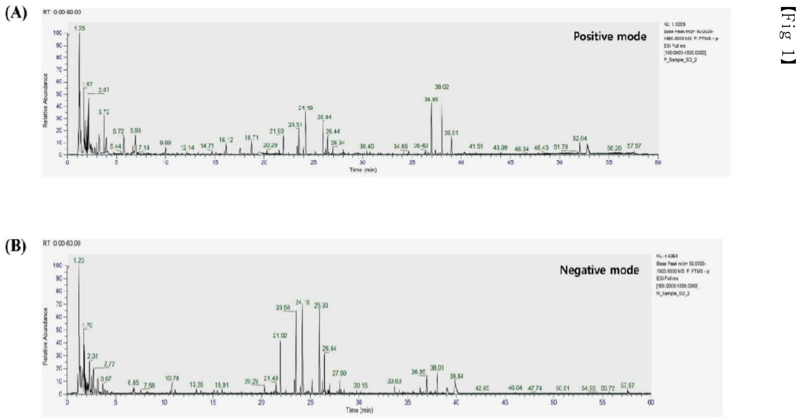

Fig. 1 is a chromatographic qualitative analysis of phenols and alkaloids in a germinated oat extract, showing (A) a positive mode and (B) a negative mode.

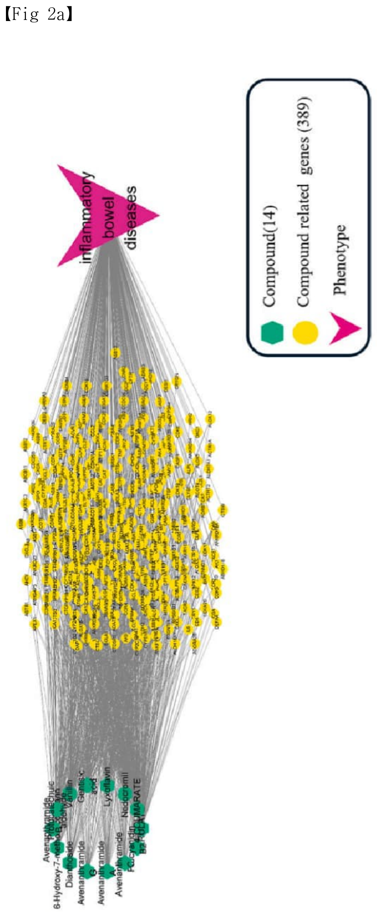

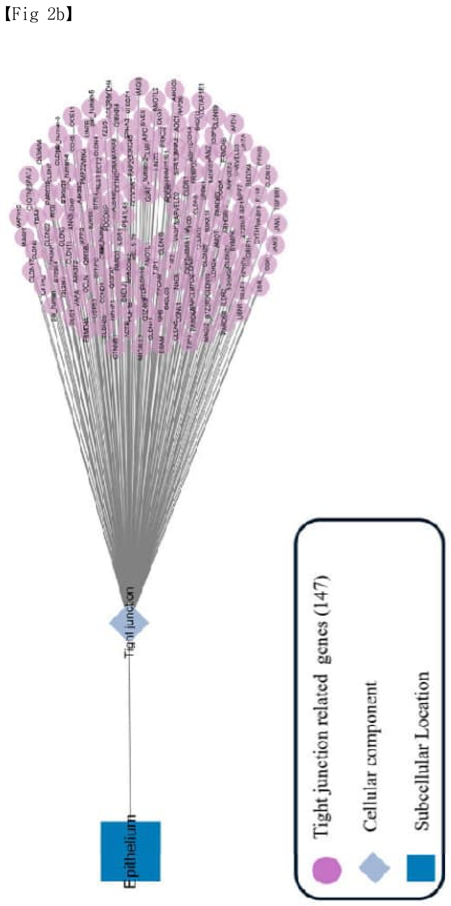

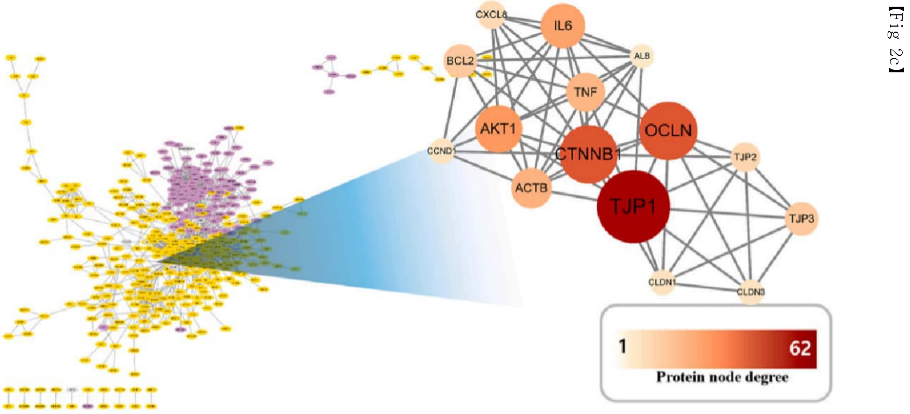

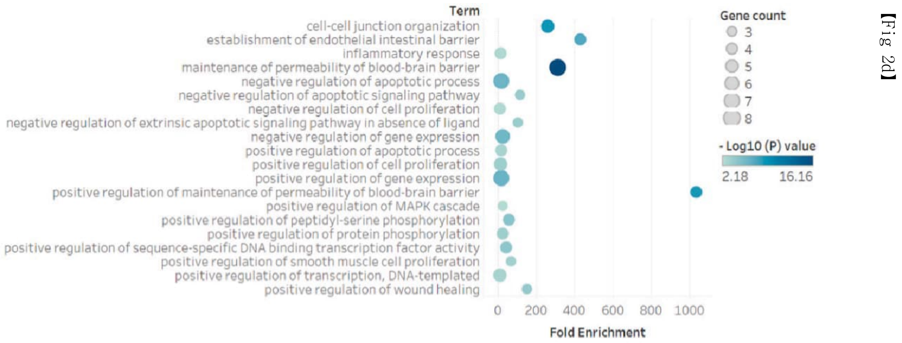

Fig. 2 illustrates the results of predictive analysis of potential target genes and biological pathways, showing: (a) a composite gene phenotype (inflammatory bowel disease) network of germinated oat; (b) tight junction (GO:CC)-related genes; (c) protein-protein interaction (PPI) analysis of a combined set of two genes from the red network of the top 15 genes identified in the PPI analysis (composite genes and tight junction genes); and (d) biological processes associated with the 15 genes analyzed through the PPI analysis.

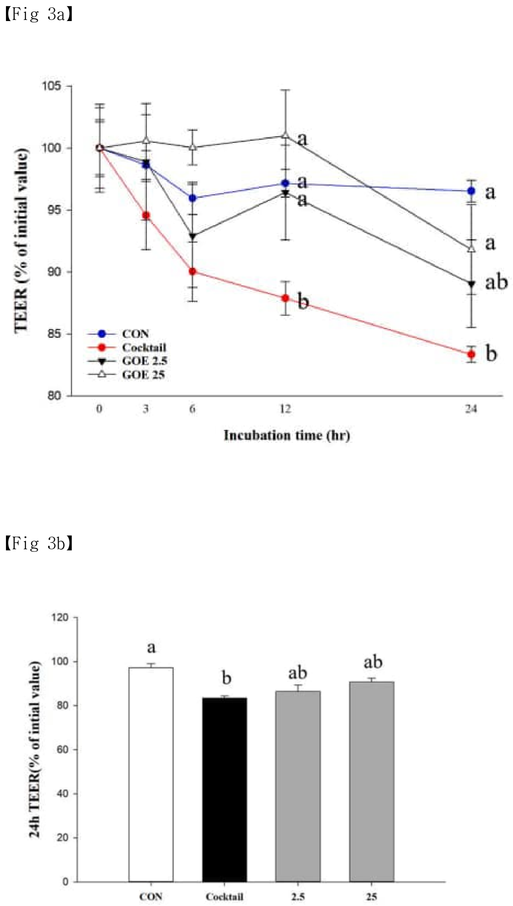

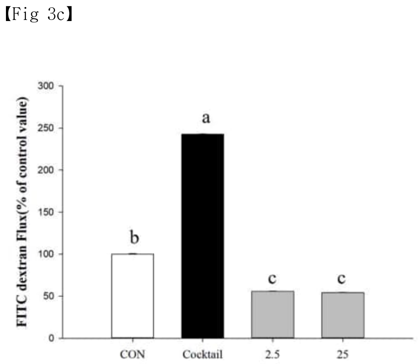

Fig. 3 illustrates the results of intestinal integrity assessment, showing: (a) changes in transepithelial electrical resistance (TEER) in Caco-2 cell monolayers after 24 hours of treatment with germinated oat; (b) comparative TEER values after 24 hours; and (c) measurement of FITC-dextran permeability. Error bars represent standard error (SE) values, and values indicated by different letters in the graphs represent significant differences according to Duncan' s multiple range test (p < 0.05).

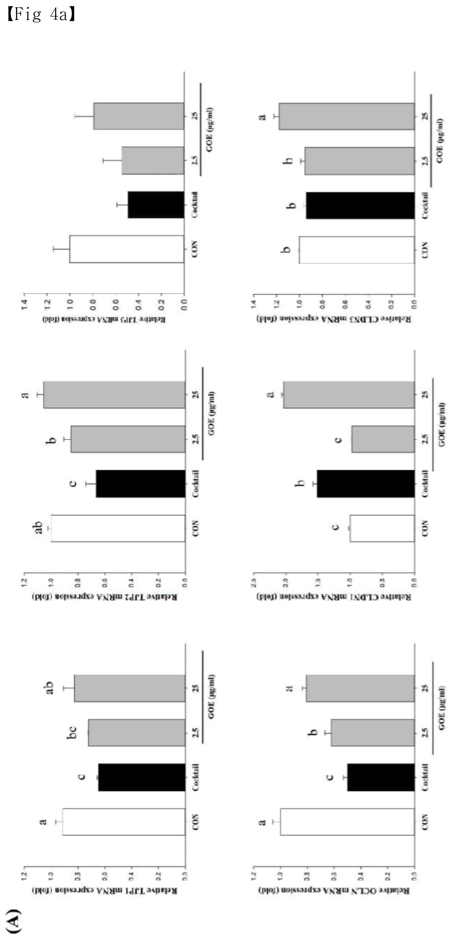

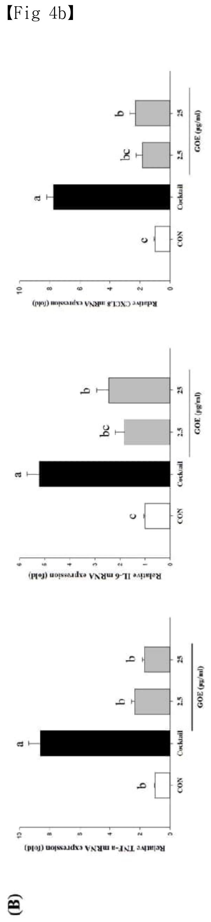

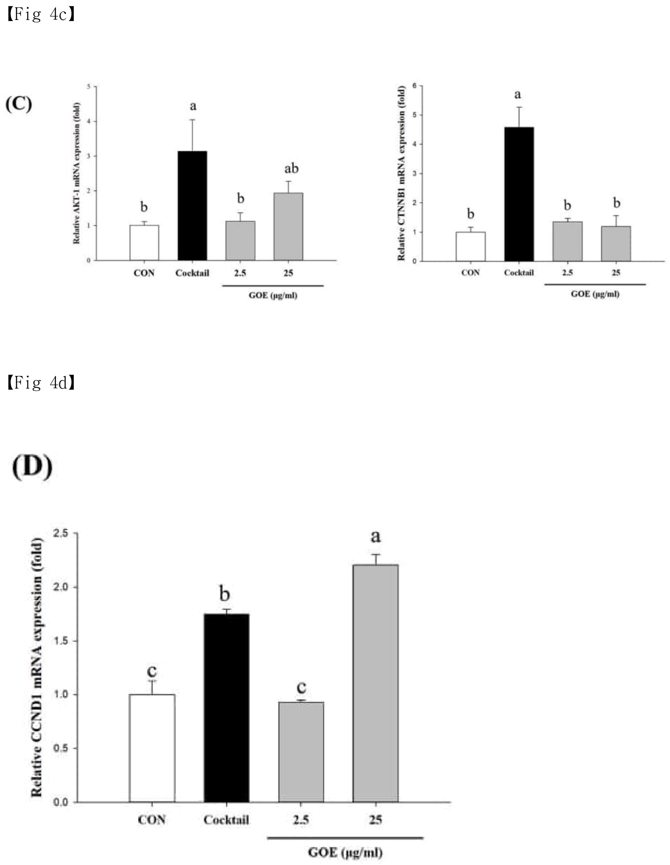

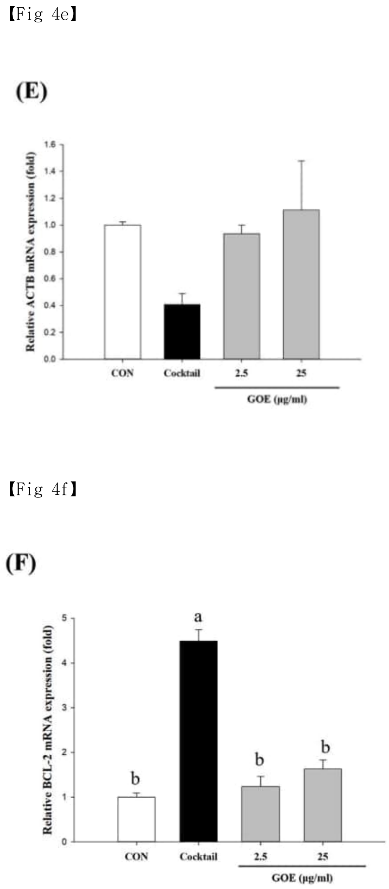

Fig. 4 illustrates the effects of germinated oat on potential inflammatory bowel disease (IBD) biomarkers, showing: (a) tight junction proteins; (b) inflammatory proteins; (c) cell signaling genes; (d) cell cycle-related genes; (e) cell structure and function; and (f) apoptotic molecules. Error bars represent standard error (SE) values, and values indicated by different letters in the graphs represent significant differences according to Duncan' s multiple range test (p < 0.05).

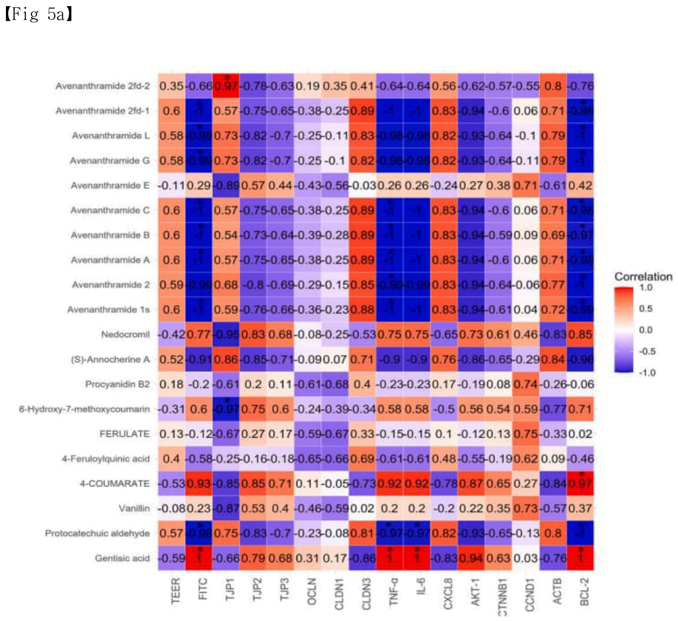

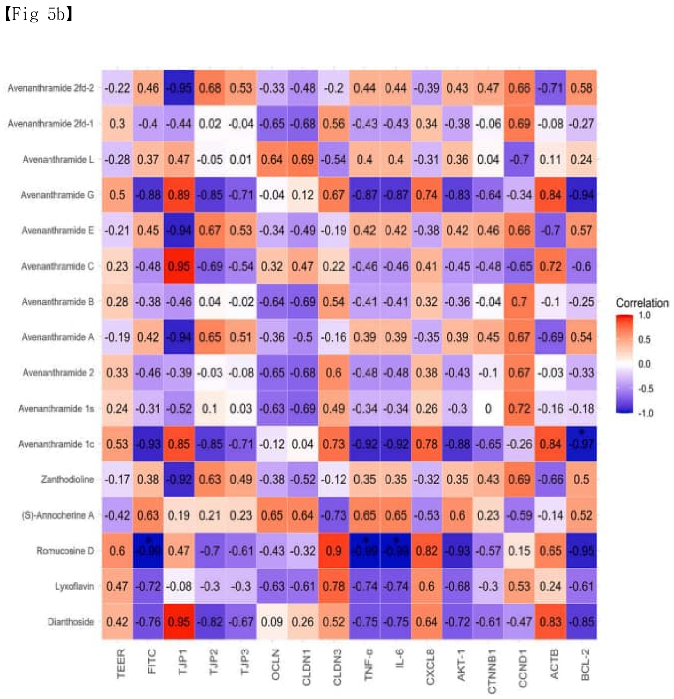

Fig. 5 illustrates the correlations between metabolites and IBD-related biomarkers, wherein each square represents a Pearson correlation coefficient value (r). Red indicates a positive correlation (0 < r < 1), blue indicates a negative correlation (-1 < r < 0), and asterisks indicate significant differences (p < 0.05). (a) Correlation analysis in the negative mode of the 25 µg/mL germinated oat-treated group; and (b) correlation analysis in the positive mode of the 25 µg/mL germinated oat-treated group.

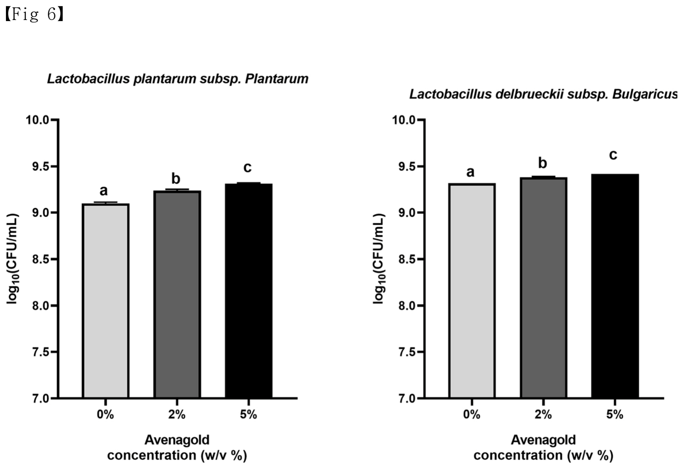

Fig. 6 is a graph illustrating the experimental results confirming that the germinated oat extract promotes the growth of lactic acid bacteria according to one embodiment of the present invention.

### [Detailed Description of the Invention]

Unless otherwise defined, all terms (including technical and scientific terms) used herein may be interpreted as having meanings commonly understood by those of ordinary skill in the art to which the present invention pertains. In addition, terms that are generally defined in dictionaries are not to be interpreted in an idealized or overly restrictive sense unless explicitly defined otherwise.

As used herein, the singular forms may include the plural forms unless the context clearly indicates otherwise.

As used herein, when a part of the specification refers to a component as being "comprising," this indicates that, unless expressly stated to the contrary, the inclusion of other components is not excluded, and additional components may also be included.

In addition, unless otherwise specified, all numerical values and expressions indicating the amounts of components, reaction conditions, and the like described in the present specification are to be understood as being modified in all instances by the term "about."

As used herein, the term "pharmaceutically acceptable" refers to a substance that, when administered in a typical therapeutic dose, can be approved for use in animals, and more specifically in humans, by a governmental or equivalent regulatory agency by avoiding significant toxic effects, is listed in a pharmacopoeia, or is otherwise generally recognized as being pharmaceutically acceptable.

As used in the present invention, the term "prevention" refers to inhibiting the occurrence of a disease or disorder in a subject who has never been diagnosed with the disease or disorder but is susceptible thereto. The term "treatment" as used herein refers to suppressing the progression of a disease or disorder, alleviating the disease or disorder, and/or eliminating the disease or disorder. The term "alleviation" as used herein refers to mitigating symptoms, suppressing the manifestation of such symptoms, delaying the manifestation thereof, or eliminating the manifestation thereof.

In addition, unless otherwise specified, the experimental procedures described in the present specification are the same as those conventionally carried out in the art.

Hereinafter, the present invention will be described in detail.

In one aspect, the present invention provides a pharmaceutical composition for preventing or treating inflammatory bowel disease (IBD), comprising a germinated oat (Avena sativa L.) extract.

As used herein, the term "germinated oat" may refer to an oat sprout having an average length of 3 to 6 mm, specifically 4 to 5 mm, and more specifically 4.2 to 4.7 mm, but is not limited thereto.

In one embodiment of the present invention, the germinated oat may be obtained by germinating oat seeds at room temperature under a 2:1 light/dark cycle for 2 days, followed by treatment with an inducer under the same light conditions for 3 days, wherein the inducer may be abscisic acid, methyl jasmonate, or a combination thereof, but is not limited thereto.

The germinated oat extract, which is the active ingredient of the present invention, may be prepared by a method comprising the following steps, but is not limited thereto:
1) extracting germinated oat with an extraction solvent; and
2) filtering the extract obtained in step 1).

In the present invention, the germinated oat used in step 1) may be either cultivated or commercially available without limitation.

In the present invention, it is preferable that the extraction in step 1) be performed using at least one solvent selected from the group consisting of water and organic solvents, and more preferably, the organic solvent is at least one selected from the group consisting of C1-C5 alcohols, ethyl acetate, acetone, ether, chloroform, benzene, hexane, and dichloromethane. The alcohol may be selected from the group consisting of methanol, ethanol, propanol, butanol, and isopropanol, and preferably is ethanol. The extraction method may include hot water extraction, ultrasonic extraction, shaking extraction, Soxhlet extraction, or reflux extraction, and specifically may be performed by hot water extraction, but is not limited thereto. In addition, the extraction time may be 1 to 24 hours, specifically 2 to 10 hours, 2 to 9 hours, or 2 to 8 hours, but is not limited thereto. Furthermore, the number of extractions may be 1 to 5 times, but is not limited thereto.

In addition, the germinated oat extract of the present invention may include a fraction obtained by re-fractionating a primary extract, which has been extracted using the extraction solvent, with another solvent having a different polarity. For example, the germinated oat extract may be a fraction obtained by extracting active components contained in germinated oat with a C1-C5 alcohol, followed by re-fractionating with a solvent having a different polarity such as ether, benzene, or hexane.

In the fractionation, two or more solvents may be used, and the solvents may be used sequentially according to their polarity or in combination to prepare respective solvent extracts, but are not limited thereto.

The extract prepared through the above process or the fraction obtained by performing the above fractionation process may subsequently be subjected to filtration, concentration, or drying to remove the solvent, and may also be subjected to all of filtration, concentration, and drying. Specifically, the filtration may be performed using a filter paper or a vacuum filter; the concentration may be performed under reduced pressure using, for example, a rotary evaporator; and the drying may be performed, for example, by spray-drying.

In the present invention, the germinated oat extract may be included at a concentration of 5 to 500 µg/ml, specifically 10 to 400 µg/ml, more specifically 15 to 300 µg/ml, even more specifically 20 to 200 µg/ml, and still more specifically 25 to 100 µg/ml, but is not limited thereto.

As used herein, the term "avenanthramides (AVNs)" refers to alkaloid compounds known to be predominantly produced in oats. The avenanthramides may be at least one selected from the group consisting of avenanthramide A, avenanthramide B, avenanthramide C, avenanthramide 0, and avenanthramide P. Specifically, the avenanthramides may refer to one or more components selected from the group consisting of avenanthramide A, avenanthramide B, and avenanthramide C, but are not limited thereto.

The avenanthramides may also be referred to as anthranilic acid amides and may be represented by the following [Chemical Formula I]:

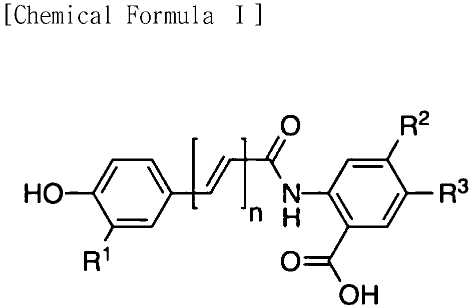

In the above chemical formula, the compound in which n = 1, R¹ is H, R² is OH, and R³ is H may be referred to as "avenanthramide A" ; the compound in which n = 1, R¹ is OCH₃ , R² is OH, and R³ is H may be referred to as "avenanthramide B" ; and the compound in which n = 1, R¹ is OH, R² is OH, and R³ is H may be referred to as "avenanthramide C."

As used herein, the term "inflammatory bowel disease (IBD)" may refer to at least one selected from the group consisting of ulcerative colitis (UC), Crohn' s disease (CD), irritable bowel syndrome, intestinal Behcet' s disease, indeterminate colitis, infectious enteritis, bacterial enteritis, viral enteritis, amoebic enteritis, hemorrhagic rectal ulcer, ischemic bowel disease, radiation enteritis, tuberculous enteritis, and leaky gut syndrome.

In one embodiment of the present invention, the inventors confirmed that the germinated oat extract improves intestinal permeability and tight junction (TJ) integrity impaired by inflammatory bowel disease, and therefore, the extract can be very usefully employed as an active ingredient of a pharmaceutical composition for preventing or treating inflammatory bowel disease.

Furthermore, according to one embodiment of the present invention, the germinated oat extract can promote the proliferation of beneficial intestinal bacteria, and more specifically, it was confirmed to improve the intestinal environment by promoting the growth of one or more lactic acid bacteria selected from the group consisting of *Lactobacillus plantarum* and *Lactobacillus delbrueckii.* Therefore, the germinated oat extract can be usefully employed as an active ingredient in a pharmaceutical composition for the prevention or treatment of inflammatory bowel disease.

The pharmaceutical composition according to the present invention may further comprise a pharmaceutically acceptable carrier in addition to the active ingredient such as the germinated oat extract.

The carrier may be one that is conventionally used in the formulation of pharmaceutically acceptable ingredients, and may include, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil. The pharmaceutical composition of the present invention may further comprise, in addition to the above ingredients, lubricants, wetting agents, sweeteners, flavoring agents, emulsifiers, suspending agents, and preservatives. Suitable pharmaceutically acceptable carriers and formulations are described in detail in Remington' s Pharmaceutical Sciences (19th ed., 1995).

In addition, an appropriate dosage of the pharmaceutical composition according to the present invention may be prescribed in various ways depending on factors such as formulation method, mode of administration, age, body weight, sex, pathological condition, diet, time of administration, route of administration, excretion rate, and responsiveness.

In addition, the pharmaceutical composition of the present invention may be administered orally or parenterally. When administered parenterally, the administration may be carried out by intravenous injection, subcutaneous injection, intramuscular injection, intraperitoneal injection, or transdermal administration. Preferably, the route of administration is determined according to the type of disease to be treated.

In addition, the pharmaceutical composition according to the present invention may be prepared in unit dosage form or filled in a multi-dose container by formulating with a pharmaceutically acceptable carrier and/or excipient, according to methods readily implementable by those skilled in the art. The formulation may be in the form of a solution, suspension, or emulsion in an oily or aqueous medium, or in the form of an extract, powder, granule, tablet, or capsule, and may additionally include a dispersing agent or a stabilizer.

In addition, the pharmaceutical composition according to the present invention may further comprise carriers and vehicles conventionally used in the pharmaceutical field. Specifically, the carriers and vehicles may include, but are not limited to, ion-exchange resins, alumina, aluminum stearate, lecithin, serum proteins (e.g., human serum albumin), buffering agents (e.g., various phosphates, glycine, sorbic acid, potassium sorbate, partial glyceride mixtures of saturated vegetable fatty acids), water, salts or electrolytes (e.g., protamine sulfate, disodium phosphate, dipotassium phosphate, sodium chloride, and zinc salts), colloidal silica, magnesium trisilicate, polyvinylpyrrolidone, cellulose substrates, polyethylene glycol, sodium carboxymethylcellulose, polyarylate, wax, or wool fat.

In addition, the pharmaceutical composition according to the present invention may be in the form of granules, powders, coated tablets, tablets, capsules, suppositories, syrups, juices, suspensions, emulsions, drops, injections, or sustained-release formulations of the active compound, and may be administered in various oral or parenteral dosage forms. When formulated, the composition may be prepared using diluents or excipients such as fillers, extenders, binders, wetting agents, disintegrants, or surfactants conventionally used in the pharmaceutical field.

In another aspect, the present invention provides a health functional food composition for preventing or alleviating inflammatory bowel disease (IBD), comprising a germinated oat (Avena sativa L.) extract.

In the present invention, the health functional food composition may contain 0.0001 to 100% by weight of the germinated oat extract, but is not limited thereto.

In addition, since the extraction method of the germinated oat extract and the types of inflammatory bowel disease are identical to those described in the pharmaceutical composition for preventing or treating inflammatory bowel disease comprising the germinated oat extract as an active ingredient, the detailed description thereof is incorporated herein by reference, and only the specific constitution of the health functional food will be described hereinafter.

In one embodiment of the present invention, the inventors confirmed that the germinated oat extract improves intestinal permeability and tight junction (TJ) integrity impaired by inflammatory bowel disease, and therefore, the extract can be very usefully employed as an active ingredient of a health functional food composition for preventing or alleviating inflammatory bowel disease.

Furthermore, according to one embodiment of the present invention, the germinated oat extract can promote the proliferation of beneficial intestinal bacteria, and specifically, it was confirmed to improve the intestinal microbial balance by promoting the growth of one or more lactic acid bacteria selected from the group consisting of *Lactobacillus plantarum* and *Lactobacillus delbrueckii.* Therefore, the germinated oat extract can be usefully employed as an active ingredient in a health functional food composition for the prevention or improvement of inflammatory bowel disease.

The health functional food composition of the present invention may be manufactured by methods conventionally used in the relevant technical field, and during the manufacture, raw materials and ingredients conventionally added in the relevant technical field may be incorporated.

In addition, the health functional food of the present invention may be used by directly adding the extract as it is or in combination with other foods or food ingredients, and may be appropriately utilized according to conventional methods.

The health functional food may be in one or more dosage forms selected from the group consisting of tablets, capsules, pills, granules, liquids, powders, flakes, pastes, syrups, gels, jellies, bars, beverages, gums, and candies, but is not limited thereto.

In still another aspect, the present invention provides a food composition for preventing or alleviating inflammatory bowel disease (IBD), comprising a germinated oat (*Avena sativa L.*) extract.

There is no particular limitation on the type of food. Examples of the food include drinks, meat, sausages, bread, biscuits, rice cakes, chocolates, candies, snacks, confectionery, pizza, ramen, other noodle products, gums, dairy products including ice cream, various soups, beverages, alcoholic drinks, and vitamin complexes, and encompass all health foods in the conventional sense.

The composition may further comprise food-acceptable additives in addition to the active ingredient, and the amount of the active ingredient mixed therein may be suitably determined according to the intended use, such as prevention, health maintenance, or therapeutic treatment.

The content of the extract according to the present invention may be suitably determined depending on its intended use (for prevention or alleviation). In general, the amount of the extract in a health food may be added in an amount of 0.01 to 15% by weight based on the total weight of the food. However, in cases of long-term intake for the purpose of health or hygiene, or for health regulation, the amount may be below the above range, and since there is no safety issue, the active ingredient may also be used in an amount exceeding the above range.

The health functional beverage composition of the present invention is not particularly limited in components other than containing the extract as an essential ingredient in the indicated proportion, and may further comprise various flavoring agents or natural carbohydrates as additional ingredients, similar to conventional beverages. Examples of the natural carbohydrates include common sugars such as monosaccharides (e.g., glucose, fructose), disaccharides (e.g., maltose, sucrose), and polysaccharides (e.g., dextrin, cyclodextrin), as well as sugar alcohols such as xylitol, sorbitol, and erythritol. In addition to the above, natural flavoring agents such as thaumatin, stevia extracts (e.g., rebaudioside A, glycyrrhizin), and synthetic flavoring agents such as saccharin and aspartame may advantageously be used.

In addition, the food of the present invention may contain various nutrients, vitamins, minerals (electrolytes), flavoring agents such as synthetic and natural flavoring agents, colorants, bulk agents (e.g., cheese, chocolate), pectic acid and salts thereof, alginic acid and salts thereof, organic acids, protective colloid thickeners, pH regulators, stabilizers, preservatives, glycerin, alcohol, and carbonating agents used in carbonated beverages. Furthermore, the extract of the present invention may contain fruit pulp for the preparation of natural fruit juices, fruit juice beverages, and vegetable beverages. These components may be used independently or in combination. Although the ratio of such additives is not particularly critical, it is generally selected in the range of more than 0 to about 20 parts by weight, based on 100 parts by weight of the extract of the present invention.

Another aspect of the present invention provides a prebiotic composition for improving intestinal microbiota, comprising a germinated oat (*Avena sativa L.*) extract as an active ingredient.

The term "for improving intestinal microbiota" refers to promoting the proliferation or growth of beneficial intestinal bacteria while suppressing the proliferation or growth of harmful intestinal bacteria, thereby maintaining a balance between beneficial and harmful microorganisms in the gut.

The term "beneficial intestinal bacteria" refers to microorganisms residing in the intestine that exert beneficial effects on the human body. For example, the beneficial intestinal bacteria may include probiotics.

The term "harmful intestinal bacteria" refers to microorganisms residing in the intestine that exert adverse effects on the human body, such as causing enteritis, and may include *Escherichia coli, Clostridium* species, and *Staphylococcus* species.

In the present invention, the term "probiotics" refers to microorganisms that exert beneficial effects on health within the body. The probiotics may include, for example, microorganisms belonging to the genera *Lactobacillus, Lactococcus, Streptococcus, Enterococcus, Pediococcus,* and *Bifidobacterium bifidum.* More specifically, the probiotics may include one or more selected from the group consisting of *Lactobacillus plantarum* and *Lactobacillus delbrueckii,* and even more specifically, one or more selected from the group consisting of *Lactobacillus plantarum subsp. plantarum* and *Lactobacillus delbrueckii subsp. bulgaricus.*

In the present invention, the term "prebiotics" refers to components that are utilized by microorganisms, including beneficial bacteria, to promote their growth or activity, thereby exerting a positive effect on the health of the host.

The present invention, in order to confirm the therapeutic effect of the germinated oat extract on inflammatory bowel disease, employed network biology and cheminformatics approaches to predict target biomarkers and molecular mechanisms, and the predicted biomarkers were validated for their effects using a cellular model of intestinal inflammation.

The effect of the germinated oat extract was verified through in vitro studies, which demonstrated a significant increase in transepithelial electrical resistance (TEER) and a decrease in fluorescein isothiocyanate (FITC) permeability. Analysis of mRNA expression of IBD-related biomarkers in Caco-2 cells showed that the mRNA levels of tight junction proteins, including TJP1, TJP2, occludin, and claudin-1, were significantly increased compared to the inflammation group. Furthermore, the germinated oat extract markedly reduced the mRNA expression levels of inflammatory cytokines such as TNF-α, IL-6, and CXCL8. By combining COCONUT with chemical profiling analyses, the fundamental molecular mechanisms of the germinated oat extract were identified. These results systematically confirm the efficacy of food components using big data-based network biology, and the germinated oat extract of the present invention can be utilized for the prevention or treatment of IBD.

Hereinafter, the present invention will be described in further detail with reference to the following Examples and Experimental Examples. However, the following Examples and Experimental Examples are provided only for illustrative purposes, and the scope of the present invention is not limited thereto.

### <Example 1> Preparation of Germinated 0at Extract

The germinated oat extract powder was obtained from Imagine the Next Green Revolution, Inc. (INGR, Yongin, Korea), where germinated oats enriched in avenanthramides were cultivated in a smart farm using molecular switch technology.

Specifically, oat seeds (*Avena sativa* cv. Daeyang) were germinated at 25° C under a 16 h:8 h light-dark cycle for 2 days, and then treated with the inducers abscisic acid (ABA) and methyl jasmonate (MJ) for 3 days at 22° C under the same light conditions. The germinated oats were harvested, washed, and hot-air dried at 40° C for one day, followed by a first hot water extraction at 120° C for 5 hours. After the first extraction, the residue was rehydrated with distilled water and subjected to a second extraction at 105° C for 3 hours. Both the first and second extracts were treated at 75° C for 1 hour with 0.2% (v/v) 1,4-alpha-D-glucan glucohydrolase (BAN 480 LS, Novozymes), followed by the addition of 0.2% (v/v) amylase (Termamyl 2X, Novozymes) and further reacted at 95° C for 1 hour. The enzymes were inactivated by heating at 105° C for 1 hour, and the mixture was filtered through a 1-µm filter, concentrated to more than 20 Brix at 65° C, and finally spray-dried using a spray dryer (SPRAY DRYER FS-2.0D, Fine ST Co.). The spray-dried germinated oat powder was dissolved in 30% ethanol solution for subsequent in vitro experiments.

### <Experimental Example 1> Profiling and Qualitative Analysis of Phenolic Compounds of Germinated Oats and Quantitative Evaluation of Avenanthramides

### Analytical Method

### Combination of high-performance liquid chromatography and Q Exactive Orbitrap mass spectrometry (HPLC/Q Exactive Orbitrap MS)

The qualitative analysis of the germinated oat extract obtained in Example 1 was performed using a Thermo Vanquish system (Thermo Fisher, Waltham, MA, USA). For chromatographic analysis, a Waters Cortects T3 column (2.1 mm × 150 mm, 1.6 µm particle size; Waters) was used.

Chromatographic analysis was performed by gradient elution at a flow rate of 0.25 mL/min using mobile phase A (water containing 0.1% formic acid) and mobile phase B (acetonitrile containing 0.1% formic acid).

The mass spectrometry conditions were as follows:
ion source type, heated electrospray ionization (H-ESI); positive ion mode voltage, 3.5 kV; negative ion mode voltage, 3 kV; sheath gas flow rate, 50 (arbitrary units, Arb); auxiliary gas flow rate, 10 (arbitrary units, Arb); sweep gas flow rate, 1 (arbitrary units, Arb); ion transfer tube temperature, 320° C.

The mass spectrometer was operated with a scan range of 100-1500 m/z, and the full width at half maximum (FWHM) was set to 70,000 for MS1 and 17,500 for MS2. Data-dependent acquisition in topN mode was performed by selecting the top 10 precursor ions for fragmentation using stepped normalized collision energy (NCE) settings of 10, 30, and 50. These settings facilitate detailed analysis and identification of compounds based on isotopic distribution patterns, ion fragmentation patterns, and accurate mass measurements by utilizing advanced mass spectral libraries and analysis software.

### Results

Analysis of phenolic compounds, alkaloid chemicals, and avenanthramides of germinated oats using HPLC-MS/MS confirmed the constituents of the germinated oats by qualitative analysis, and as a result, a total of 23 compounds were identified in the spray-dried germinated oats (Fig. 1).

Table 1 shows chromatograms presenting details such as retention time, ionization mode, precursor ions, and product ions, as well as the quantitative analysis results of major constituents of the germinated oat compounds by HPLC-MS/MS. In addition, Table 2 presents the quantitative analysis results of avenanthramides contained in the spray-dried germinated oat extract, wherein each value represents the mean ± standard deviation.

**[Table 1]**

| **No** | **R.T (min)** | **Analyte Name** | **Molecular Formula** | **adduct** | **Theoretical Mass** | **Measured Mass** |
|---|---|---|---|---|---|---|
| 1 | 4.14 | Dianthoside | C 12H 160 8 | [M+Na]+ | 311.074 | 288.1 |
| 2 | 5.21 | Gentisic acid | C 7H 6O 4 | [M-H]- | 153.01933221 990947 | 154 |
| 3 | 7.63 | Protocatechuic aldehyde | C 7H 6O 3 | [M-H]- | 137.02441759 990944 | 138 |
| 4 | 13.77 | Romucosine D | C 21H 23NO 5 | [M+Na]+ | 392.14684353 60905 | 369.2 |
| 5 | 14.03 | Vanillin | C 8H 8O 3 | [M-H]- | 151.04006766 390944 | 152 |
| 6 | 15.03 | Lyxoflavin | C 17H 20N 4O 6 | [M+H]+ | 377.14556081 20905 | 376.1 |
| 7 | 15.11 | Avenanthramide 1c | C 16H 13NO 5 | [M+H]+ | 300.08664896 809057 | 299.1 |
| 8 | 15.17 | 4-COUMARATE | C 9H 8O 3 | [M+HCO2]- | 366.11944017 99095 | 164 |
| 9 | 15.86 | 4-Feruloylquinic acid | C 17H 20O 9 | [M-H]- | 367.10345576 790945 | 368.1 |
| 10 | 17.51 | 6-Hydroxy-7-methoxycoumarin | C 10H 8O 4 | [M-H]- | 191.03498228 390947 | 192 |
| 11 | 17.72 | FERULATE | C 10H 10O 4 | [M-H]- | 193.05063234 790947 | 194.1 |
| 12 | 17.97 | Avenanthramide E | C 17H 15NO 5 | [M+H]+ | 314.10229903 20905 | 313.1 |
| 13 | 19.73 | Procyanidin B2 | C 30H 26O 12 | [M+H]+ | 579.14970272 40905 | 578.1 |
| 14 | 21.92 | Avenanthramide C | C 16H 13NO 6 | [2M-H]- | 629.14129781 99094 | 629.140 |
| 15 | 23.36 | (S)-Annocherine A | C 17H 15NO 4 | [2M-H]- | 593.19293946 79095 | 593.187 |
| 16 | 23.51 | Avenanthramide A | C 16H 13NO 5 | [2M-H]- | 597.15146857 99095 | 597.150 |
| 17 | 23.98 | Zanthodioline | C 16H 19NO 5 | [M+Na]+ | 328.11554340 | 328.117 |
| | | | | | 80905 | |
| 18 | 24.18 | Avenanthramide B | C 17H 15NO 6 | [2M-H]- | 657.17259794 79095 | 657.171 |
| 19 | 24.8 | Avenanthramide 2 | C 18H 17NO 7 | [M+H]+ | 360.10777833 60905 | 359.1 |
| 20 | 25.12 | Nedocromil | C 19H 17NO 7 | [M-H]- | 370.09322543 190945 | 371.1 |
| 21 | 25.94 | Avenanthramide 1s | C 18H 17NO 6 | [M+H-H2O]+ | 326.10229903 20905 | 343.1 |
| 22 | 26.2 | Avenanthramide G | C 16H 13NO 5 | [M+H]+ | 300.08664896 809057 | 299.1 |
| 23 | 26.35 | Avenanthramide L | C 18H 15NO 5 | [M+H]+ | 326.10229903 209057 | 325.1 |
| 24 | 26.44 | Avenanthramide 2fd-1 | C 19H 17NO 6 | [M+Na]+ | 378.09480796 409053 | 355.1 |
| 25 | 26.81 | Avenanthramide 2fd-2 | C 19H 17NO 6 | [M+H]+ | 356.11286371 609054 | 355.1 |

**[Table 2]**

| **Compound** | **Content (mg/kg)** |
|---|---|
| Avenanthramide A | 2648.21 ± 117.14 |
| Avenanthramide B | 4643.577 ± 81.53 |
| Avenanthramide C | 2752.36 ± 50.68 |

In the negative mode, gentisic acid, protocatechuic aldehyde, vanillin, 4-coumarate, 4-feruloylquinic acid, 6-hydroxy-7-methoxycoumarin, ferulate, procyanidin B2, and nedocromil were detected, whereas in the positive mode, dianthoside, romucosine D, lyxoflavin, avenanthramide 1c, and zanthodioline were identified. In addition, AVN A, AVN B, AVN C, AVN E, AVN G, AVN 2, AVN 1s, AVN L, AVN 2fd-1, AVN 2fd-2, and (S)-Annocherine A were detected in both negative and positive modes.

### <Experimental Example 2> In Silico Analysis - Gene-Phenotype Network Approach

The in silico experiment was used to predict potentially important biomarkers that could be regulated by germinated oats.

### 2-1. Comprehensive Analysis of Tight Junction Genes Regulated by Germinated 0at Compounds

Based on the compound profiling of Experimental Example 1, the effect of germinated oats on intestinal health functions was analyzed.

### Selection of Test Gene Sets

To accurately identify tight junction and germinated oat-related plant genes, network analysis was performed using the COCONUT database and Quick Gene Ontology (GO). Compound-gene association data and gene-phenotype association data were collected using COCONUT. COCONUT provides a standardized, structured, and integrated database of natural products, which is used to explore components, associated efficacies, and target genes, and to investigate diseases that may be affected by gene alterations induced by herbal compounds.

Tight junction-related genes were collected using Gene Ontology (GO) annotations, and the genes were specifically obtained from the "cellular component" category focusing on tight junction-related genes.

Among the 25 compounds of germinated oats, 14 compounds were identified as major compounds associated with IBD.

The two-layered network consisted of 404 nodes and 1,220 edges (Fig. 2a). The focus was placed on genes potentially regulated by compounds identified in germinated oats, with particular emphasis on identifying genes associated with intestinal health. A total of 389 genes were mapped to the compound-gene-phenotype network scaffold (Fig. 2a). To identify specific functions related to intestinal barrier health, tight junction-related genes in *Homo sapiens* were examined. Genes associated with intestinal permeability and tight junctions were selected from the Quick GO cellular component (CC) category, and a total of 147 *Homo sapiens* genes associated with tight junction function were specifically identified and integrated into a gene set, as shown in Fig. 2b.

### 2-2. Identification of Interactions Between Germinated 0at-Derived Target Genes and Tight Junction Genes

The focus was placed on identifying genes potentially regulated by compounds contained in germinated oats, particularly those related to the maintenance and function of tight junctions, which are critical for intestinal health. To confirm the relationship between tight junction-related genes and compound-driven genes, two gene datasets were combined, and a protein-protein interaction (PPI) network analysis was performed on the combined dataset.

### Analytical Method

Using the above datasets, a PPI analysis was performed to identify potential biomarkers associated with both compound-related genes and tight junction genes. For the PPI analysis, compound-driven phenotype-related genes were combined with tight junction-related genes, and the analysis was conducted using the combined gene set. The STRING database (version 12.0) was employed for the protein-protein interaction network analysis. All genes were mapped to *Homo sapiens* genes, and Cytoscape (version 3.10.0) was used for visualization.

### Results

Fifteen genes were identified in the order of JP1, CTNNB1, OCLN, AKT1, IL6, ACTB, TNF, BCL2, TJP3, TJP2, CXCL8, CCND1, CLDN1, CLDN3, and ALB (Fig. 2c).

The PPI analysis classified the genes shown in Table 3 according to their functions, and as a result, six functional categories were identified for the classified genes: tight junction, cell signaling, cell cycle, inflammatory response, apoptosis, and cell structure and function.

**[Table 3]**

| **No** | **Gene symbol** | **Annotation** | **Protein node degree** | **DB source** | **Function** |
|---|---|---|---|---|---|
| 1 | TJP1 | Tight junction protein 1, ZO-1 | 62 | Quick GO | Tight junction |
| 2 | CTNNB1 | Catenin beta-1 | 51 | Quick GO | Cell signaling |
| 3 | OCLN | Occludin | 51 | Quick GO | Tight junction |
| 4 | AKT1 | RAC-alpha serine/threonine-protein kinase | 43 | COCONUT | Cell signaling |
| 5 | ACTB | Actin, cytoplasmic 1 | 38 | Quick GO | Cell structure and function |
| 6 | TJP3 | Tight junction protein 3 | 33 | Quick GO | Tight junction |
| 7 | TJP2 | Tight junction protein 2 | 30 | Quick GO | Tight junction |
| 8 | CLDN1 | Claudin-1 | 27 | Quick GO | Tight junction |
| 9 | CLDN3 | Claudin-3 | 27 | Quick GO | Tight junction |
| 10 | CCND1 | G1/S-specific cyclin-D1 | 27 | COCONUT | Cell cycle |
| 11 | IL6 | Interleukin-6 | 41 | COCONUT | Inflammatory |
| 12 | TNF | Tumor necrosis factor | 37 | COCONUT | Inflammatory |
| 13 | BCL2 | Apoptosis regulator Bcl-2 | 33 | COCONUT | Cell apoptosis |
| 14 | CXCL8 | C-X-C motif chemokine ligand 8 | 29 | COCONUT | Inflammatory |
| 15 | ALB | Serum albumin | 26 | COCONUT | Other |

### 2-3. Prediction of Biological Pathways for Inflammatory Response and Intestinal Barrier Integrity Genes in IBD Through Gene Ontology Analysis

### Analytical Method

To elucidate the mechanism by which the germinated oat extract promotes intestinal health in Caco-2 cells, biological pathway analysis was performed using DAVID (https://david.ncifcrf.gov/tools.jsp). Among the genes identified in the PPI analysis, the top 15 genes were used for pathway prediction analysis, and the results were expressed according to gene ontology biological processes. To identify biological pathways associated with inflammatory bowel disease (IBD), only IBD-related pathways were selected. For the selection of IBD-related biological pathways, relevant keywords associated with IBD were collected through the Human Phenotype Ontology (https://hpo.jax.org/app/, Gargano et al., 2023).

Phenotypic keyword searches using Quick GO were employed to identify biological pathways associated with the above terms. Subsequently, a sequential analysis was performed focusing on the top 15 genes derived from Quick GO and the list of biological pathways, with Human Phenotype Ontology (HPO) keywords used as references. Only biological pathways that overlapped between the analyses were presented as results, and visualization was performed using Tableau (version 2022.2.0). All pathway results were considered significant at p < 0.05.

### Results

Gene ontology enrichment analysis related to IBD identified significant biological processes including inflammatory response, intracellular signaling, positive regulation of gene expression, negative regulation of apoptotic processes, and both positive and negative regulation of cell proliferation. Processes such as "inflammatory response," "cell-cell junction organization," "positive regulation of gene expression," "positive regulation of wound healing," "maintenance of blood-brain barrier permeability," and "establishment of endothelial intestinal barrier" were among those ranked (Fig. 2d).

The associations between the genes identified through the PPI analysis and specific biological processes, as well as detailed statistical significance values, are presented in Table 4. All biological processes shown in Table 4 exhibit statistical significance with p-values of less than 0.05.

**[Table 4]**

| **Category** | **Term** | **List Total** | **Count** | **Genes** | **Fold Enrichment** | **P-Value** | **FDR** |
|---|---|---|---|---|---|---|---|
| GO_BP | maintenance of permeability of blood-brain barrier | 15 | 8 | IL6, TJP2, OCLN, CLDN3, TJP3, TJP1, ACTB, CLDN1 | 314.3919192 | 6.95198E-17 | 5.02E-14 |
| GO_BP | positive regulation of gene expression | 15 | 7 | IL6, CTNNB1, OCLN, TNF, CLDN3, CXCL8, AKT1 | 17.49145793 | 8.77087E-07 | 0.000114 |
| GO_BP | negative regulation of apoptotic process | 15 | 7 | IL6, BLC2, ALB, CTNNB1, TNF, AKT1, TJP1 | 16.81123457 | 1.10564E-06 | 0.000114 |
| GO_BP | negative regulation of gene expression | 15 | 6 | CTNNB1, OCLN, TNF, CLDN3, CXCL8, AKT1 | 24.39247649 | 2.03778E-06 | 0.000168 |
| GO_BP | cell-cell junction organization | 15 | 5 | TJP2, OCLN, TJP3, TJP1, CLDN1 | 259.3733333 | 2.1046E-09 | 7.14E-07 |
| GO_BP | positive regulation of cell | 15 | 5 | IL6, BLC2, AKT1, TIP1, ACTB | 11.89785933 | 0.000487629 | 0.020553 |
| GO_BP | proliferation positive regulation of transcription, DNA-templated | 15 | 5 | IL6, CTNNB1, TNF, AKT1, ACTB | 8.907051282 | 0.001442174 | 0.04165 |
| GO_BP | positive regulation of maintenance of permeability of blood-brain barrier | 15 | 4 | TJP2, OCLN, TJP3, TJP1 | 1037.493333 | 2.96477E-09 | 7.14E-07 |
| GO_BP | establishment of endothelial intestinal barrier | 15 | 4 | TJP2, TJP3, TJP1, CLDN1 | 432.2888889 | 6.50316E-08 | 1.17E-05 |
| GO_BP | positive regulation of peptidyl-serine phosphorylation | 15 | 4 | IL6, BLC2, TNF, AKT1 | 55.77921147 | 3.7062E-05 | 0.00223 |
| GO_BP | positive regulation of sequence-specific DNA binding | 15 | 4 | IL6, CTNNB1, TNF, AKT1 | 43.96158192 | 7.54276E-05 | 0.004189 |
| | transcription factor activity | | | | | | |
| GO_BP | positive regulation of protein phosphorylation | 15 | 4 | CCND1, TNF, CLDN3, AKT1 | 22.95339233 | 0.000512392 | 0.020553 |
| GO_BP | positive regulation of apoptotic process | 15 | 4 | IL6, BLC2, CTNNB1, TNF | 15.43888889 | 0.001613832 | 0.044815 |
| GO_BP | inflammatory response | 15 | 4 | IL6, TNF, CXCL8, AKT1 | 11.89785933 | 0.003385982 | 0.08731 |
| GO_BP | negative regulation of cell proliferation | 15 | 4 | IL6, CTNNB1, CLDN3, CXCL8 | 11.22828283 | 0.003985699 | 0.098108 |
| GO_BP | positive regulation of wound healing | 15 | 3 | OCLN, CLDN3, CLDN1 | 149.6384615 | 0.000154781 | 0.007982 |
| GO_BP | negative regulation of apoptotic signaling pathway | 15 | 3 | BLC2, CTNNB1, TNF | 114.4294118 | 0.000266298 | 0.012818 |
| GO_BP | negative regulation of extrinsic | 15 | 3 | BLC2, TNF, AKT1 | 105.1513514 | 0.00031575 | 0.014248 |
| | apoptotic signaling pathway in absence of ligand | | | | | | |
| GO_BP | positive regulation of smooth muscle cell proliferation | 15 | 3 | IL6, TNF, AKT1 | 67.07931034 | 0.000776948 | 0.028048 |
| GO_BP | positive regulation of MAPK cascade | 15 | 3 | IL6, CTNNB1, TNF | 22.61976744 | 0.006595958 | 0.12211 |
| GO_BP | positive regulation of cell migration | 15 | 3 | CLDN3, TJP1, CLDN1 | 14.57153558 | 0.015318828 | 0.22258 |
| GO_BP | regulation of membrane permeability | 15 | 2 | TJP2, CLDN3 | 432.2888889 | 0.004310891 | 0.098108 |
| GO_BP | protein localization to adherens junction | 15 | 2 | TJP1, ACTB | 432.2888889 | 0.004310891 | 0.098108 |
| GO_BP | regulation of transmembrane transporter activity | 15 | 2 | BLC2, ACTB | 370.5333333 | 0.005027693 | 0.098108 |
| GO_BP | positive regulation of bicellular tight junction assembly | 15 | 2 | CLDN3, CLDN1 | 370.5333333 | 0.005027693 | 0.098108 |
| GO_BP | inflammatory response to wounding | 15 | 2 | IL6, TNF | 370.5333333 | 0.005027693 | 0.098108 |
| GO_BP | positive regulation of I-kappaB phosphorylation | 15 | 2 | TNF, AKT1 | 370.5333333 | 0.005027693 | 0.098108 |
| GO_BP | negative regulation of lipid storage | 15 | 2 | IL6, TNF | 288.1925926 | 0.00645986 | 0.12211 |
| GO_BP | positive regulation of leukocyte adhesion to vascular endothelial cell | 15 | 2 | IL6, TNF | 185.2666667 | 0.010031906 | 0.172453 |
| GO_BP | positive regulation of cyclin-dependent protein | 15 | 2 | CCND1, AKT1 | 129.6866667 | 0.014302619 | 0.22258 |
| | serine/threonine kinase activity | | | | | | |
| GO_BP | positive regulation of protein localization to cell surface | 15 | 2 | TNF, AKT1 | 123.5111111 | 0.015012739 | 0.22258 |
| GO_BP | positive regulation of glial cell proliferation | 15 | 2 | IL6, TNF | 117.8969697 | 0.015722384 | 0.22258 |
| GO_BP | regulation of myelination | 15 | 2 | CTNNB1, AKT1 | 117.8969697 | 0.015722384 | 0.22258 |
| GO_BP | negative regulation of neurogenesis | 15 | 2 | IL6, TNF | 108.0722222 | 0.01714025 | 0.237986 |
| GO_BP | positive regulation of nitric-oxide synthase activity | 15 | 2 | TNF, AKT1 | 103.7493333 | 0.017848472 | 0.238641 |
| GO_BP | negative regulation of intrinsic apoptotic signaling pathway | 15 | 2 | BLC2, AKT1 | 92.63333333 | 0.019970294 | 0.262156 |
| GO_BP | positive regulation of cytokine production involved in inflammatory response | 15 | 2 | IL6, TNF | 81.05416667 | 0.02279277 | 0.293864 |
| GO_BP | regulation of angiogenesis | 15 | 2 | IL6, CTNNB 1 | 70.1009009 | 0.02631025 | 0.321966 |
| GO_BP | positive regulation of glucose import | 15 | 2 | OCLN, AKT1 | 70.1009009 | 0.02631025 | 0.321966 |
| GO_BP | extrinsic apoptotic signaling pathway via death domain receptors | 15 | 2 | BLC2, TNF | 64.84333333 | 0.028415091 | 0.336323 |
| GO_BP | T cell differentiation in thymus | 15 | 2 | BLC2, CTNNB 1 | 64.84333333 | 0.028415091 | 0.336323 |
| GO_BP | branching involved in ureteric bud morphogenesis | 15 | 2 | BLC2, CTNNB 1 | 58.94848485 | 0.031214974 | 0.348548 |
| GO_BP | positive regulation of | 15 | 2 | IL6, TNF | 57.63851852 | 0.031913773 | 0.348548 |
| | chemokine production | | | | | | |
| GO_BP | regulation of insulin secretion | 15 | 2 | IL6, TNF | 56.38550725 | 0.032612104 | 0.348548 |
| GO_BP | positive regulation of nitric oxide biosynthetic process | 15 | 2 | TNF, AKT1 | 55.1858156 | 0.033309967 | 0.348548 |
| GO_BP | positive regulation of JAK-STAT cascade | 15 | 2 | IL6, TNF | 55.1858156 | 0.033309967 | 0.348548 |
| GO_BP | positive regulation of G1/S transition of mitotic cell cycle | 15 | 2 | CCND1, AKT1 | 50.85751634 | 0.036096748 | 0.357012 |
| GO_BP | intrinsic apoptotic signaling pathway in response to DNA damage | 15 | 2 | BLC2, TNF | 50.85751634 | 0.036096748 | 0.357012 |
| GO_BP | regulation of cyclin- | 15 | 2 | CCND1, ACTB | 48.03209877 | 0.038181935 | 0.366274 |
| | dependent protein serine/threonine kinase activity | | | | | | |
| GO_BP | regulation of G1/S transition of mitotic cell cycle | 15 | 2 | CCND1, ACTB | 48.03209877 | 0.038181935 | 0.366274 |
| GO_BP | positive regulation of epithelial to mesenchymal transition | 15 | 2 | IL6, CTNNB1 | 47.15878788 | 0.038876067 | 0.366274 |
| GO_BP | positive regulation of protein localization to plasma membrane | 15 | 2 | TNF, AKT1 | 46.31666667 | 0.039569733 | 0.366274 |
| GO_BP | negative regulation of cysteine-type endopeptidase activity involved in apoptotic process | 15 | 2 | TNF, AKT1 | 46.31666667 | 0.039569733 | 0.366274 |
| GO_BP | positive regulation of cell differentiation | 15 | 2 | CTNNB1, ACTB | 43.96158192 | 0.041647943 | 0.380631 |
| GO_BP | negative regulation of fat cell differentiation | 15 | 2 | IL6, TNF | 42.52021858 | 0.043031096 | 0.388356 |
| GO_BP | positive regulation of neuron apoptotic process | 15 | 2 | CTNNB1, TNF | 41.8344086 | 0.043721977 | 0.389719 |
| GO_BP | negative regulation of autophagy | 15 | 2 | BLC2, AKT1 | 41.17037037 | 0.044412394 | 0.391046 |
| GO_BP | positive regulation of interleukin-8 production | 15 | 2 | IL6, TNF | 39.90358974 | 0.045791842 | 0.393592 |
| GO_BP | positive regulation of interleukin-1 beta production | 15 | 2 | IL6, TNF | 38.71243781 | 0.04716944 | 0.400663 |
| GO_BP | positive regulation of tyrosine | 15 | 2 | IL6, TNF | 38.14313725 | 0.047857546 | 0.401781 |
| | phosphorylation of STAT protein | | | | | | |
| GO_BP | G1/S transition of mitotic cell cycle | 15 | 2 | CCND1, BLC2 | 37.59033816 | 0.048545191 | 0.402869 |

### <Experimental Example 3> In Vitro Experiment

To confirm that the genes predicted through the in silico analysis are regulated by treatment with germinated oats and thereby improve intestinal health, an in vitro experiment was conducted.

### Cell Culture

Caco-2 cells (passages 33-37) were purchased from ATCC (Manassas, VA, USA). The cells were cultured in Dulbecco's Modified Eagle's Medium (DMEM; Biowest, Nuaillé, Cholet, France) supplemented with 10% fetal bovine serum (FBS; Gibco BRL, NY, USA), 1% penicillin-streptomycin (P/S; Corning Inc., NY, USA), and 1% non-essential amino acids (NEAAs; Gibco, Rockville, MD, USA) at 37 ° C in a 5% CO₂ incubator. For the experiments, the cells were seeded at a density of 3.75 × 10⁵ cells/well in polyester 6-well Transwell^{®} plates (0.4 µm pore size; Costar, Kennebunk, ME, USA) and cultured for 21 days to allow differentiation. The culture medium was replaced every 2-3 days. The experimental concentrations applied were determined to be non-cytotoxic based on the results obtained from the CCK-8 assay.

### Cell Viability

Cell viability was evaluated using the Cell Counting Kit-8 (CCK-8) assay (Sigma-Aldrich), a cell proliferation assay based on the cleavage of 2-(2-methoxy-4-nitrophenyl)-3-(4-nitrophenyl)-5-(2,4-disulfophenyl)-2H-tetrazolium (WST-8).

Specifically, the cells were seeded at a density of 1 × 10⁵ cells per well in 24-well plates and cultured for 21 days to allow differentiation. After 21 days, the culture medium was replaced with serum-free medium. The cells were then treated with various concentrations of germinated oat extract (GOE) (1, 2.5, 5, 10, 25, 50, and 100 µg/mL) for 24 hours. Following the 24-hour incubation, 100 µL of CCK-8 solution was added to each well, and the cells were further incubated for 4 hours in a 5% CO₂ atmosphere at 37 ° C.

### 3-1. Evaluation of Intestinal Health Status Using TEER and FITC-Dextran

In the present invention, the focus was primarily placed on the integrity of tight junctions, which play a critical role in the pathophysiology of inflammatory bowel disease (IBD). An in vitro model reflecting intestinal inflammation observed in IBD was established using an inflammatory cytokine cocktail, and transepithelial electrical resistance (TEER) and FITC-dextran permeability assays were employed to evaluate epithelial barrier integrity and permeability.

### Transepithelial Electrical Resistance (TEER)

To evaluate the effect of the germinated oat extract on intestinal barrier integrity under inflammatory conditions, a combinatorial treatment approach was employed. Initially, a cytokine cocktail consisting of 25 ng/mL interleukin (IL)-1β, 10 ng/mL lipopolysaccharide (LPS), 50 ng/mL tumor necrosis factor (TNF)-α, and 50 ng/mL interferon (IFN)-γ was applied to the basolateral side of the Transwell system to simulate an intestinal inflammatory environment. Simultaneously, germinated oat extract at concentrations of 2.5 and 25 µg/mL, together with LPS (10 µg/mL), was introduced into the apical side in serum-free medium. Following the simultaneous application of germinated oat extract and the cytokine cocktail, TEER values were measured at 0, 3, 6, 12, and 24 hours using a Millicell^{®} ERS system (Millipore).

### Fluorescein Isothiocyanate (FITC)-Dextran Flux

Higher fluorescence intensity values indicate higher permeability. A FITC-dextran solution (100 µg/mL) together with germinated oat extract was added to the apical side, and after 24 hours of incubation, the fluorescence was collected from the basolateral side of the Transwell system. The collected fluorescent solution was measured using a SpectraMax i3x Multi-Mode Microplate Reader at excitation and emission wavelengths of 485 nm and 535 nm, respectively.

### Results

To evaluate the effect on the integrity and permeability of Caco-2 epithelial cells, transepithelial electrical resistance (TEER) and FITC-dextran permeability were measured. In the cytokine cocktail-treated group, the TEER values were found to be significantly lower than those of the control group.

The samples were treated with germinated oat extract at concentrations of 25 or 2.5 µg/mL, and significant effects were observed at 12 hours posttreatment (p < 0.05). In the germinated oat extract-treated groups, TEER values were improved in a dose-dependent manner compared with the cytokine cocktail group (Figs. 3a and 3b). In the cocktail-treated group, FITC-dextran flux increased approximately 2.5-fold compared with the control group, whereas treatment with germinated oat extract markedly reduced the FITC flux (Fig. 3c).

When treated with germinated oat extract, intestinal barrier function was markedly improved, as demonstrated by increased TEER values and decreased FITC-dextran flux. These results indicate that the bioactive components of the germinated oat extract exert protective effects against cytokine-induced barrier disruption, thereby confirming its potential role in regulating intestinal permeability in the context of IBD.

### 3-2. PCR Validation of Germinated Oat-Induced Gene Regulation

Subsequently, the potential genetic basis of these effects was analyzed, and fifteen candidate genes were identified that are associated with intestinal integrity and predicted to be influenced by germinated oat compounds, which are considered to mediate the protective effects of oats on intestinal integrity. To validate the predicted genes, PCR was performed using a Caco-2 monolayer model, focusing on various biomarkers.

### RNA Isolation and Quantitative Real-Time Reverse Transcription Polymerase Chain Reaction (qRT-PCR)

Total RNA was isolated from Caco-2 cells after 24 hours of treatment using TRIzol^{®} reagent (Life Technologies, Rockville, MD, USA) in accordance with the manufacturer' s instructions. Following treatment of the cells with TRIzol and chloroform, the resulting mixture was centrifuged at 12,000 rpm for 20 minutes at 4 ° C. The supernatant was carefully transferred, mixed with an equal volume of isopropanol, and centrifuged again under the same conditions to precipitate mRNA. The quantity of total RNA was assessed using a NanoDrop^{™} Lite Spectrophotometer. Complementary DNA (cDNA) was synthesized from total RNA using the Transcriptor First Strand cDNA Synthesis Kit (Roche).

The top 14 genes identified through protein-protein interaction (PPI) analysis were validated using qRT-PCR. The mRNA expression levels were quantified using the relative 2^{-ΔΔCT} method, normalized to the expression of the housekeeping gene GAPDH.

### Results

Among the fifteen genes analyzed by PPI, TJP1, TJP2, TJP3, OCLN, CLDN1, and CLDN3 were associated with tight junction function. Tight junction proteins TJP1, TJP2, and OCLN, which are critical for maintaining epithelial barrier integrity, were significantly decreased under the cytokine cocktail condition compared with the control group. However, when treated with germinated oat extract, TJP1, TJP2, and OCLN were significantly upregulated compared with the cocktail group (p = 0.0027, p = 0.0025, p < 0.0001).

**In** contrast, the expression of TJP3 and CLDN3, which are members of the tight junction protein family, did not change significantly in the germinated oat extract-treated groups, although a tendency toward increase was observed (Fig. 4a). TNF-α, IL-6, and CXCL8 are inflammatory cytokines upregulated in IBD; the mRNA expression levels of TNF-α, CXCL8, and IL-6 were significantly increased by approximately fourfold in the cocktail group, whereas they were markedly reduced in the germinated oat extract-treated groups (p < 0.0001) (Fig. 4b). In addition, CTNNB1 was significantly decreased in response to germinated oat extract treatment (p < 0.0001), while AKT1 did not reach statistical significance but exhibited a similar trend; both were regulated by germinated oat extract (Fig. 4c). In the case of CCND1, differential responses were observed depending on the concentration of the extract.

In the present invention, two concentrations of germinated oat extract, 2.5 µg/mL and 25 µg/mL, were employed. The results demonstrated that CCND1 expression was decreased at a concentration of 2.5 µg/mL, whereas at the higher concentration of 25 µg/mL, CCND1 expression was increased (Fig. 4d). At the same time, expression of tight junction proteins was markedly increased and levels of inflammatory cytokines were significantly reduced. These findings confirm that germinated oat extract exerts a dose-dependent effect in improving intestinal health.

The β-actin gene (ACTB), which is associated with cellular structure and function, was significantly decreased in the cytokine cocktail-treated group but was significantly increased in the germinated oat extract-treated group (Fig. 4e). In contrast, cyclin D (CCND1), which is involved in cell cycle regulation, and BCL-2, which is associated with apoptosis, were both significantly increased in the cocktail-treated group. In the germinated oat extract-treated group, the expression of BCL-2 was significantly reduced (Fig. 4f). Fourteen out of the fifteen genes were validated (Fig. 4). These results confirm that the germinated oat extract of the present invention contributes to maintaining barrier integrity by regulating the ACTB gene.

### 3-3. Correlation Analysis Between Avenanthramide-Rich Germinated Oat Compounds and IBD-Related Markers at Various Treatment Concentrations

Fig. 5 illustrates the strength of correlations among the analyzed factors, where red, blue, and white indicate positive, negative, and no correlations, respectively. Notably, avenanthramide compounds of germinated oats exhibited stronger correlations in the negative mode compared to the positive mode. TEER values generally showed positive correlations with avenanthramide compounds, whereas FITC-dextran permeability assay values demonstrated significant negative correlations with avenanthramide compounds. Among tight junction genes, TJP1 and CLDN3 exhibited the strongest positive correlations with avenanthramide compounds, while TJP2 and TJP3 showed positive correlations with other phenolic compounds, such as 4-coumarate and nedocromil, rather than avenanthramides. In the context of inflammation-related genes, TNF-α and IL-6 demonstrated significant negative correlations with phenolic compounds including avenanthramides, protocatechuic aldehyde, and romucosine D. The effect on CXCL8 showed negative correlations with non-avenanthramide phenolic compounds, such as gentisic acid and 4-coumarate. Cell signaling genes such as AKT1 and CTNNB1 generally exhibited negative correlations with avenanthramide compounds, whereas CCND1 showed positive correlations with non-avenanthramide alkaloid and phenolic compounds in the negative mode. The actin cytoskeleton gene ACTB displayed predominantly positive correlations with avenanthramide compounds, while the BCL-2 gene generally exhibited significant negative correlations with avenanthramide compounds. These results confirm specific interactions between germinated oat metabolites and key biological markers, indicating the potential therapeutic effects of the germinated oat extract on inflammatory bowel disease.

### <Experimental Example 4> Effect of Germinated Oat Extract on the Growth Promotion of Lactic Acid Bacteria

It is well known that intestinal lactic acid bacteria improve the microbial balance in the gut by producing organic acids that lower intestinal pH and inhibit the growth of harmful bacteria. Accordingly, in the present invention, the effect of the germinated oat extract on the growth of beneficial intestinal bacteria was evaluated by measuring the change in proliferation of lactic acid bacteria following treatment with the germinated oat extract. As a result, it was confirmed that the germinated oat extract of the present invention promoted the growth of lactic acid bacteria and thereby contributed to improving the intestinal environment.

### Medium Composition

MRSA + Germinated oat extract (0%, 1%, 5%)
: MRS broth agar (MRSA)
: Base medium: 50 plates (MB-P1040-P50)
: Germinated oat extract: 0%, 1%, and 5% concentrations

### Test Method - Standard Plate Method

① Dilute 10-25 g (mL) of the sample with diluent at a 1:9 ratio (further dilution performed as needed).
② Dispense 1 mL of each dilution into two Petri dishes (KS-P0101).
③ Pour MRSA medium, previously cooled to 43-45° C, into each dish, mix thoroughly, and allow it to solidify. Once solidified, overlay with an additional 3-5 mL of the same medium.
④ Incubate at 30 ± 1° C for 24 hours.

Specifically, lactic acid bacteria *Lactobacillus plantarum subsp. plantarum* and *Lactobacillus delbrueckii subsp. bulgaricus* were inoculated into MRS media supplemented with the germinated oat extract prepared in Example 1 at concentrations of 2% and 5%. The inoculation was carried out by adding 1% of the bacterial suspension (10⁹ CFU/mL) to achieve a final concentration of 10⁷ CFU/mL. The cultures were incubated aerobically at 30° C for 24 hours in an incubator, after which changes in the total viable counts of lactic acid bacteria were measured. The viable cell counts were determined according to the standard plate count method described in the Korean Food Code, Chapter 8, General Test Methods - Section 4.5.1 ( "General Bacterial Count" ). Specifically, 10-25 g (mL) of each sample was diluted with diluent at a 1:9 ratio, and 1 mL of each dilution was spread onto two Petri dishes (KS-P0101). The number of lactic acid bacteria (CFU/mL) was then calculated. As a control, MRS medium containing 0% germinated oat extract was used, and bacterial counts were determined in the same manner. The results are shown in Table 5 and Fig. 6 below.

**[Table 5]**

| Germinated oat extract (w/v %) | Strains | |
|---|---|---|
| | *Lactobacillus plantarum* subsp. *Plantarum* (log₁₀CFU/mL) | *Lactobacillus delbrueckii* subsp. *Bulgaricus* (log₁₀CFU/mL) |
| 0 | 9.10±0.01^{A} | 9.32±0.11^{A} |
| 2 | 9.25±0.05^{B} | 9.39±0.10^{B} |
| 5 | 9.32±0.01^{C} | 9.42±0.10^{C} |

Data are expressed as the mean ± SD (n = 2). Values with different uppercase letters within the same column are significantly different (P < 0.05) according to Tukey' s range test. CFU, colony-forming unit.

As a result, both *Lactobacillus plantarum* and *Lactobacillus delbrueckii* showed a significant increase in the groups treated with the germinated oat extract (2% and 5%) compared to the control group (0%).

### Statistical Analysis

Data were expressed as mean ± standard error (SE). Statistical evaluation was performed using one-way analysis of variance (ANOVA) with SAS 9.4 software (SAS Institute, Cary, NC, USA). For post-hoc analysis, Duncan' s multiple range test was employed for both TEER and FITC-dextran measurements as well as PCR data analysis. Statistical significance for comparisons with a p-value of less than 0.05 was indicated by different letters assigned to each group.

In conclusion, the experimental validation of the germinated oat extract demonstrated positive regulation of permeability and effective modulation of nine out of fourteen genes examined (TJP1, TJP2, OCLN, CLDN1, TNF, IL-6, CXCL8, CTNNB1, and CCND1). In particular, the germinated oat extract effectively regulated genes associated with tight junctions and inflammation. In addition, the germinated oat extract was found to promote the growth of beneficial intestinal bacteria (particularly lactic acid bacteria), thereby improving the balance of the intestinal microbiota. Accordingly, the germinated oat extract of the present invention can be effectively used for the prevention, amelioration, or treatment of inflammatory bowel diseases by maintaining the integrity of tight junctions between intestinal epithelial cells, regulating intestinal permeability and inflammatory responses, and simultaneously enhancing the growth of beneficial intestinal microorganisms.

The foregoing description of the present invention is intended for illustrative purposes only, and it will be understood by those skilled in the art that various modifications and alterations can be readily made without departing from the spirit or essential characteristics of the present invention. Therefore, the embodiments described above should be understood as exemplary in all respects and not as limiting.

## Claims

1. A pharmaceutical composition for use in the prevention or treatment of inflammatory bowel disease (IBD), comprising a germinated oat (Avena sativa L.) extract and a pharmaceutically acceptable carrier.

2. The pharmaceutical composition for use according to claim 1, wherein the inflammatory bowel disease is at least one selected from the group consisting of ulcerative colitis (UC), Crohn' s disease (CD), irritable bowel syndrome, intestinal Behcet' s disease, indeterminate colitis, infectious enteritis, bacterial enteritis, viral enteritis, amoebic enteritis, hemorrhagic rectal ulcer, ischemic bowel disease, radiation enteritis, tuberculous enteritis, and leaky gut syndrome.

3. The pharmaceutical composition for use according to claim 1 or 2, wherein the germinated oat is obtained by germinating oat seeds under a light-dark cycle of 2:1 for 1 to 3 days, followed by treatment with an inducer under a light-dark cycle of 2:1 for 2 to 5 days.

4. The pharmaceutical composition for use according to claim 3, wherein the inducer is abscisic acid, methyl jasmonate, or a combination thereof.

5. The pharmaceutical composition for use according to any one of claims 1 to 4, wherein the germinated oat extract is prepared by a method comprising:
1) extracting germinated oat with an extraction solvent; and
2) filtering the extract obtained in step 1), and concentrating or drying the filtered extract,
wherein the extraction solvent of step 1) is at least one solvent selected from the group consisting of water and an organic solvent, and wherein the drying of step 2) is performed by spray-drying.

6. The pharmaceutical composition for use according to claim 5, wherein the organic solvent is at least one selected from the group consisting of a C1-C5 alcohol, ethyl acetate, acetone, ether, chloroform, benzene, hexane, and dichloromethane.

7. The pharmaceutical composition for use according to any one of claims 1 to 6, wherein the germinated oat extract is **characterized by** improving intestinal permeability and tight junction (TJ) integrity impaired by inflammatory bowel disease.

8. The pharmaceutical composition for use according to any one of claims 1 to 6, wherein the germinated oat extract promotes the growth of beneficial intestinal microorganisms.

9. The pharmaceutical composition for use according to claim 8, wherein the beneficial microorganism is at least one lactic acid bacterium selected from the group consisting of *Lactobacillus plantarum* and *Lactobacillus delbrueckii.*

10. A health functional food composition for the prevention or alleviation of inflammatory bowel disease (IBD), comprising a germinated oat (Avena sativa L.) extract.

11. The health functional food composition according to claim 10, wherein the inflammatory bowel disease is at least one selected from the group consisting of ulcerative colitis (UC), Crohn' s disease (CD), irritable bowel syndrome, intestinal Behcet' s disease, indeterminate colitis, infectious enteritis, bacterial enteritis, viral enteritis, amoebic enteritis, hemorrhagic rectal ulcer, ischemic bowel disease, radiation enteritis, tuberculous enteritis, and leaky gut syndrome.

12. The health functional food composition according to claim 10 or 11, wherein the germinated oat extract is **characterized by** improving intestinal permeability and tight junction (TJ) integrity impaired by inflammatory bowel disease.

13. The health functional food composition according to any one of claims 10 to 12, wherein the health functional food is in a formulation selected from the group consisting of tablets, capsules, pills, granules, liquids, powders, flakes, pastes, syrups, gels, jellies, bars, beverages, gums, and candies.

14. A food composition for the prevention or alleviation of inflammatory bowel disease (IBD), comprising a germinated oat (Avena sativa L.) extract.

15. A prebiotic composition for improving intestinal microbiota, comprising a germinated oat (Avena sativa L.) extract as an active ingredient.
